# EUROPEAN PATENT SPECIFICATION

(11) **EP 0 827 756 B1**
(45) Date of publication and mention of the grant of the patent: **02.07.2003**
(21) Application number: 97202763.5
(22) Date of filing: 09.09.1997
(51) Int. Cl.: A61M 25/00

(54) **Catheter with internal stiffening ridges**
Katheter mit internen Versteifungsgegenständen
Cathéter avec nervures de rigidité internes

(30) Priority: 09.09.1996 NL 1003984
(43) Date of publication of application: 11.03.1998
(73) Proprietor: CORDIS EUROPA N.V., NL-9301 LJ Roden (NL)
(72) Inventor: Mouris-Laan, Judith Alida Maria Petronella, 8604 CN Sneek (NL); Kasto, Lesley Sugito, 9469 PN Schipborg (NL)
(74) Representative: 't Jong, Bastiaan Jacob

(56) References cited:
- EP-A- 0 454 264
- WO-A-86/00232
- WO-A-93/08864
- DE-A- 2 820 239
- DE-A- 4 113 265
- US-A- 5 593 394

## Description

The invention relates to a catheter comprising a tube-like basic body which is formed by a first tube-like element with a lumen which is bounded by a lumen wall and at least a second tube-like element which has been received in the lumen and comprises an outer wall, wherein of the outer wall and the lumen wall at least one has been provided with a number of ridges substantially extending in the longitudinal direction of the basic body.

Such a catheter is known from DE-A-2 820 239. The ridges reinforce in the first place the tube-like element on which they have been arranged, so that it does not buckle so easily. In addition, when bending the catheter the ridges may rest against the other tube-like element, which furthermore reduces the chance of buckling of the tube-like element onto which the ridges have been arranged. Thirdly, also the tube-like element which itself has not been provided with ridges, but is positioned against the ridges of the other tube-like element, becomes less prone to buckling due to the support provided in this manner. The basic body of the catheter according to the invention has consequently a very considerable buckling resistance.

According to the invention the measure as set out in the characterizing portion of claim 1 is employed, leading to a simple construction of the basic body. The second tube-like element may be pushed inside the first tube-like element and does not need to be fixed inside it. The favourable mutual influence of the buckling behaviour of the separate tube-like elements is also achieved in this case, as, because of the flattening deformation of the tube-like elements, a good contact is achieved between the ridges and the opposite wall on bending.

An advantageous further development has been characterised in claim 3. The top-surface supports the opposite wall evenly over its entire surface area, without concentrations of stress occurring which expedite buckling.

A high buckling resistance is achieved with the measures as set out in claim 4.

A suitable embodiment, which is also suitable for small to very small catheter-diameters, has additionally been characterised in claim 5.

In order to prevent a preferred bending direction of the basic body, the measure as set out in claim 6 is preferably employed.

The invention will be explained in greater detail in the following description with reference to the attached drawings.

Figure 1 shows a perspective view of a balloon catheter according to the invention.

Figure 2 shows a cross-section along the line II-II of figure 1.

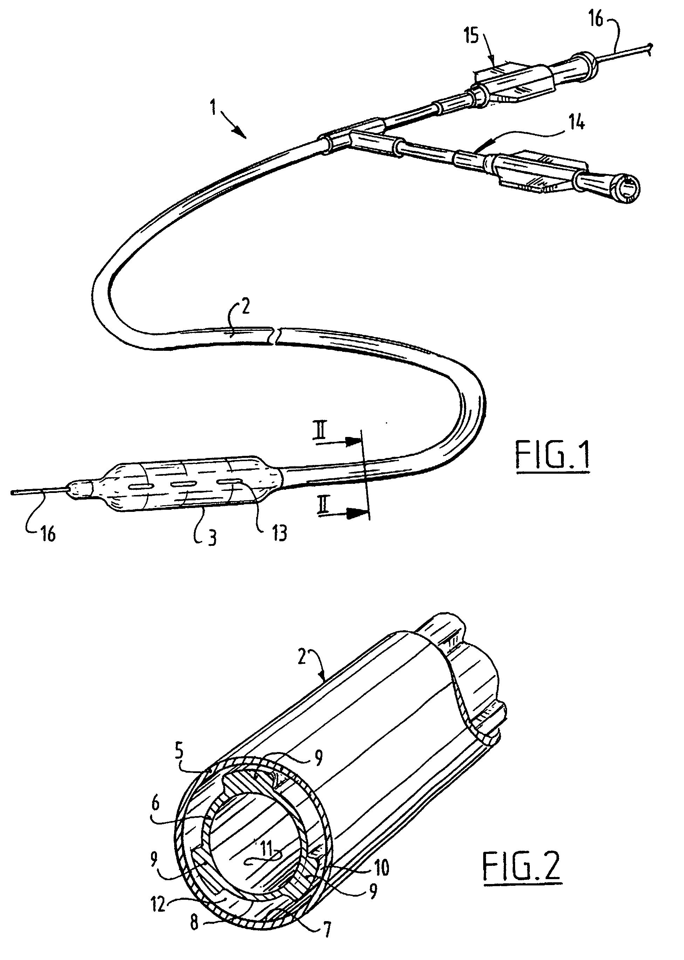

A balloon catheter 1 illustrated in figure 1 comprises a tube-like basic body 2 at a distal end of which a balloon member 3 has been arranged. The proximal end of the basic body 2 has been connected to connecting members 14, 15, the functions of which will be explained in greater detail below.

As can be seen in figure 2, the basic body 2 has been formed by a first tube-like element 5 with a lumen 12 which is bounded by a lumen wall 7. A second tube-like element 6 has been received inside the lumen 12, which itself comprises a lumen 11.

With this example of an embodiment the outer wall 8 of the second tube-like element 6 has been provided with ridges 9 which extend in the longitudinal direction of the basic body 2. The ridges 9 reinforce the second tube-like element 6 to such an extent that it tends to buckle less easily.

On bending the basic body 2 rather sharply, the lumen wall 7 will be positioned against the top-surfaces 10 of the ridges 9 due to a flattening of the cross-section of the first tube-like element 5. On the one hand the second tube-like element 6 is supported as a result, so that it can be bent more sharply before it buckles. On the other hand also the first tube-like element 5 is supported internally by the ridges 9, so that also this first tube-like element 5 buckles less quickly. The result of these effects is that the basic body 2 of the catheter 1 displays a significant resistance to buckling.

As can be seen in figure 2, the top-surfaces 10 of the ridges 9 are substantially concentric with the tube-like element 6 and consequently also with the tube-like element 5, so that these top-surfaces 10 make contact with the lumen wall 7 in an even fashion. Consequently, on making contact no concentrations of stress will occur in the wall of the first tube-like element 5, which is favourable in order to prevent buckling.

The function of the central lumen 11 in the second tube-like element 6 of catheter 1 is to accommodate a guide wire 16.

As is known, first a guide wire is introduced into a patient after which a catheter such as the catheter 1 is passed over the guide wire 16 into the body of the patient, until the balloon member 3 has arrived in the target position. The second tube-like element 6 extends over the entire length of the catheter and the lumen 11 thereof is accessible via the connecting member 15 at the proximal end.

The remaining space of the lumen 12 of the first tube-like element 5 has been made accessible via a connecting member 14 of which a channel is connected to this lumen 12 via an opening in the wall of the first tube-like element 5. At the distal end a number of openings 13 have been arranged in the wall of the first tube-like element 5 at the site of the balloon member 3, so that the lumen 12 is connected with the inside of the balloon member 3 via these openings 13. By introducing fluid under pressure into the balloon member 3 via the connecting member 14, this balloon member 3 may be expanded in order to carry out for instance angioplasty.

Although in the embodiment illustrated the ridges 9 have been arranged on the outer wall of the second, inner tube-like element 6, it is also possible to provide the first, outer tube-like element 5 on the inside, on the lumen wall 7, with ridges. These ridges will reinforce in that case the first tube-like element, and due to the cooperation with the second tube-like element as described above, the resistance to buckling of the entire basic body 2 is furthermore increased.

In a suitable manner the number of ridges 9 is three, and the ridges have been arranged evenly distributed around the circumference. Other quantities of ridges are possible as well however.

In this way many variations of the inventive idea are possible. All these variations are considered to fall within the scope of the attached claims.

## Claims

1. Catheter 1 comprising a tube-like basic body 2 which is formed by a first tube-like element 5 with a lumen 12 which is bounded by a lumen wall 7 and at least a second tube-like element 6 which has been received in the lumen 12 and comprises an outer wall 8, wherein of the outer wall 8 and the lumen wall at least one has been provided with a number of ridges 9 substantially extending in the longitudinal direction of the basic body 2, **characterized in that** the second tube-like element 6 has been received in the lumen 12 of the first tube-like element 5 in a loose manner.

2. Catheter as claimed in claim 1, wherein a first connecting member 15 is coupled to the proximal end of the catheter 1 and has a channel which communicates with a lumen 11 of the second tube-like element 6, and a second connecting member 14 is coupled to the proximal end of the catheter 1 and has a channel which communicates with a lumen 12 defined between the inside wall of the first tube-like element 5 and the outside wall of the second tube-like element 6.

3. Catheter as claimed in claim 1, wherein the ridges 9 have a top-surface which is substantially concentric with the tube-like element 6 carrying the ridges.

4. Catheter as claimed in claim 1, wherein the number of ridges is odd and the ridges 9 have been arranged evenly distributed around the circumference.

5. Catheter as claimed in claim 4, wherein the number of ridges 9 is three.

6. Catheter as claimed in claim 1, wherein the ridges extend in a helical pattern.

7. Catheter as claimed in one of the preceding claims, comprising a balloon member 3 arranged at the distal end of the tube-like basic body 2, a number of openings 13 having been arranged in the wall of the first tube-like element 5 at the side of the balloon member 3, so that the lumen 12 is connected with the inside of the balloon member 3 via these openings 13.

## Patentansprüche

1. Katheter 1 mit einem röhrenförmigen Grundkörper 2, der durch ein erstes röhrenförmiges Element 5 mit einem Lumen 12, welches von einer Lumenwand 7 begrenzt ist, und mindestens ein zweites röhrenförmiges Element 6, welches im Lumen 12 aufgenommen worden ist und eine Außenwand 8 umfasst, gebildet ist, wobei von der Außenwand 8 und der Lumenwand mindestens eine mit einer Anzahl von Rippen 9 versehen worden ist, die sich im Wesentlichen in der Längsrichtung des Grundkörpers 2 erstrecken, **dadurch gekennzeichnet, dass** das röhrenförmige Element 6 auf eine lockere Weise im Lumen 12 des ersten röhrenförmigen Elements 5 aufgenommen worden ist.

2. Katheter wie im Anspruch 1 beansprucht, wobei ein erstes Verbindungselement 15 mit dem proximalen Ende des Katheters 1 gekoppelt ist und einen Kanal aufweist, der mit dem Lumen 11 des zweiten röhrenförmigen Elements 6 in Verbindung steht, und ein zweites Verbindungselement 14 mit dem proximalen Ende des Katheters 1 gekoppelt ist und einen Kanal aufweist, der mit einem Lumen 12, welches zwischen der Innenwand des ersten röhrenförmigen Elements 5 und der Außenwand des zweiten röhrenförmigen Elements 6 begrenzt ist, in Verbindung steht.

3. Katheter wie im Anspruch 1 beansprucht, wobei die Rippen 9 eine obere Oberfläche aufweisen, die im Wesentlichen konzentrisch ist mit dem die Rippen tragenden röhrenförmigen Element 6.

4. Katheter wie im Anspruch 1 beansprucht, wobei die Anzahl der Rippen ungeradzahlig ist und die Rippen 9 gleichmäßig verteilt um den Umfang herum angeordnet worden sind.

5. Katheter wie im Anspruch 4 beansprucht, wobei die Anzahl der Rippen 9 drei beträgt.

6. Katheter wie im Anspruch 1 beansprucht, wobei die Rippen sich in einem helikalen Muster erstrecken.

7. Katheter wie in einem der vorangehenden Ansprüche beansprucht, mit einem Ballonelement 3, das beim distalen Ende des röhrenförmigen Grundkörpers angeordnet ist, einer Anzahl von Öffnungen 13, die in der Wand des ersten röhrenförmigen Elements 5 an der Stelle des Ballonelements 3 angeordnet wurden, so dass das Lumen 12 über diese Öffnungen 13 mit dem Inneren des Ballonelements verbunden ist.

## Revendications

1. Cathéter 1 comprenant un corps de base semblable à un tube 2 qui est formé par un premier élément semblable à un tube 5 avec une lumière 12 qui est limitée par une paroi de lumière 7 et au moins un second élément semblable à un tube 6 qui est reçu dans la lumière 12 et qui comprend une paroi extérieure 8, dans lequel un élément au moins parmi la paroi extérieure 8 et la paroi de lumière est muni d'un certain nombre de stries 9 s'étendant sensiblement dans la direction longitudinale du corps de base 2, **caractérisé en ce que** le second élément semblable à un tube 6 est reçu dans la lumière 12 du premier élément semblable à un tube 5 de manière lâche.

2. Cathéter selon la revendication 1, dans lequel un premier élément de connexion 15 est couplé à l'extrémité proximale du cathéter 1 et possède un canal qui communique avec une lumière 11 du second élément semblable à un tube 6, et dans lequel un second élément de connexion 14 est couplé à l'extrémité proximale du cathéter 1 et possède un canal qui communique avec une lumière 12 définie entre la paroi intérieure du premier élément semblable à un tube 5 et la paroi extérieure du second élément semblable à un tube 6.

3. Cathéter selon la revendication 1, dans lequel les stries 9 possèdent une surface supérieure qui est sensiblement concentrique avec l'élément semblable à un tube 6 portant les stries.

4. Cathéter selon la revendication 1, dans lequel le nombre de stries est impair et les stries 9 sont réparties de manière régulière autour de la circonférence.

5. Cathéter selon la revendication 4, dans lequel le nombre de stries 9 est de trois.

6. Cathéter selon la revendication 1, dans lequel les stries s'étendent selon un schéma hélicoïdal.

7. Cathéter selon l'une quelconque des revendications précédentes, comprenant un élément formant ballon 3 disposé au niveau de l'extrémité distale du corps de base semblable à un tube 2, un certain nombre d'ouvertures 13 ayant été disposées dans la paroi du premier élément semblable à un tube 5 du côté de l'élément formant ballon 3, de sorte que la lumière 12 soit connectée avec l'intérieur de l'élément formant ballon 3 via ces ouvertures 13.
